(19) Europäisches Patentamt
European Patent Office
Office européen des brevets

(11) Numéro de publication: **0 348 257 A2**

(12) # DEMANDE DE BREVET EUROPEEN

(21) Numéro de dépôt: **89401445.5**

(22) Date de dépôt: **26.05.89**

(51) Int. Cl.4: **C 07 D 257/04**
C 07 D 271/10,
C 07 D 285/12,
C 07 D 401/04,
C 07 D 403/06,
C 07 C 121/75,
C 07 C 133/00,
C 07 C 161/00, A 61 K 31/41

(30) Priorité: **27.05.88 FR 8807114**

(43) Date de publication de la demande:
**27.12.89 Bulletin 89/52**

(84) Etats contractants désignés:
**AT BE CH DE ES FR GB GR IT LI LU NL SE**

(71) Demandeur: **DELALANDE S.A.**
**32, rue Henri Regnault**
**F-92400 Courbevoie (FR)**

(72) Inventeur: **Milcent, René**
**6, rue Cavendich**
**F-75019 Paris (FR)**

**Lebreton, Luc**
**2, rue Pierre Loti Bât B.**
**F-78600 Maisons Laffitte (FR)**

**Mazouz, Fathi**
**5, avenue du Docteur Tenine**
**F-92160 Antony (FR)**

**Burstein, Claude**
**21, avenue du Bois Brandin**
**F-60580 Coye la Forêt (FR)**

**Gueddari, Salah**
**5, chemin des Bas Heurts**
**F-93160 Noisy le Grand (FR)**

(74) Mandataire: **Kedinger, Jean-Paul et al**
**c/o Cabinet Malemont 42, avenue du Président Wilson**
**F-75116 Paris (FR)**

Revendications pour les Etats contractants suivants: ES + GR.

(54) **Nouveaux dérivés d'(hétéro)aryl diazole, leur procédé de prépation et leur application en thérapeutique.**

(57) Composés à usage thérapeutique de formule

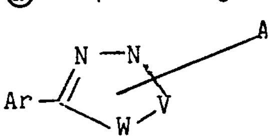

(I)

où l'enchaînement -W-V-N- représente -N=N-N-, -N-N=N-, -O-CO-N-, -O-CS-N-, -S-CO-N- ou -S-CS-N- ; Ar est un groupe aryle ; et A est un groupe vinyle ou une chaîne alkylène terminée par CN, méthoxy, éthoxy, OH, halogène, $NH_2$ éventuellement substitué.

EP 0 348 257 A2

## Description

### Nouveaux dérivés d'(hétéro)aryl diazole, leur procédé de préparation et leur application en thérapeutique

La présente invention a pour objet de nouveaux dérivés d'(hétéro)aryl diazole, leur procédé de préparation et leur application en thérapeutique.

Les dérivés selon l'invention répondent plus précisément à la formule :

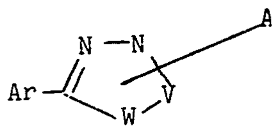

(I)

dans laquelle l'enchaînement

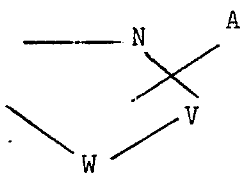

représente

1) soit l'enchaînement de structure :

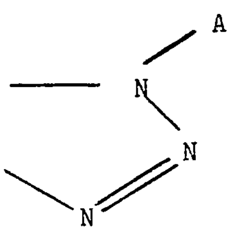

ou

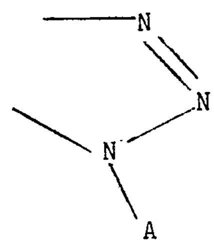

où A est :

a) un groupe vinyle ou un groupe $(CH_2)_n$-$Z_1$ dans laquelle $Z_1$ représente un groupe CN, méthoxy ou éthoxy et n est un nombre entier de 1 à 6, auquel cas Ar prend la valeur $Ar_1$ qui représente :

. un noyau phényle,

. un noyau phényle substitué par un ou deux atomes d'halogène ou un groupe $NO_2$, méthyle, éthyle, méthoxy, éthoxy, phényle ou benzyloxy de formule :

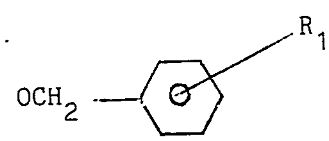

où $R_1$ représente un atome d 'hydrogène, un ou deux atome d'halogène ou un groupe CN, $NO_2$, $NH_2$, $CF_3$, alkyle en $C_1$-$C_4$, alkoxy en $C_1$-$C_4$ ou acylamino en $C_2$-$C_4$, ou

. un noyau hétérocyclique ; ou

b) un groupe de formule $(CH_2)_m$-$Z_2$ dans laquelle $Z_2$ représente un groupe OH, un atome d'halogène, un groupe $NH_2$, un groupe $NH_2$ substitué par un ou deux groupes alkyle en $C_1$-$C_4$, un groupe acylamino en $C_2$-$C_4$ ou un groupe phtalimido et m est un nombre entier de 2 à 6, auquel cas Ar prend la valeur $Ar_2$ qui représente :

. un noyau phényle substitué par un groupe alkoxy en $C_1$-$C_4$ ou benzyloxy de formule

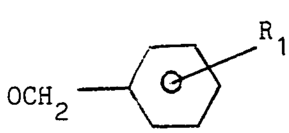

où $R_1$ a la même signification que ci-dessus, ou

. un noyau hétérocyclique ;

2) soit l'enchaînement de structure

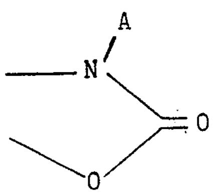 , 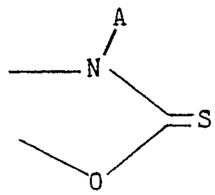 , 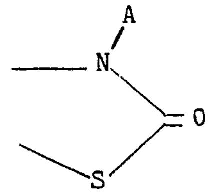 ou 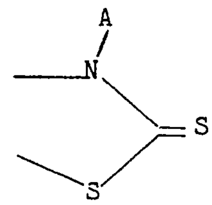

où A est un groupe cyano-2 éthyle, auquel cas Ar prend la valeur $Ar_3$ qui représente un noyau phényle substitué par un groupe alkyle en $C_1$-$C_4$, alkoxy en $C_1$-$C_4$, phényle ou benzyloxy de formule

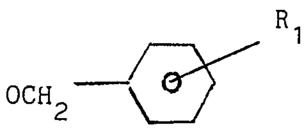

où $R_1$ a la même signification que ci-dessus, ainsi que les sels d'addition d'acide de ces dérivés (I) comportant un groupe salifiable, à l'exclusion du phényl-5 tétrazole-2 butyronitrile et du phényl-5 tétrazole-2 valéronitrile

Il est à noter que dans ce qui précède et ce qui suit, l'expression "alkyle en $C_1$-$C_4$" vise les groupes méthyle, éthyle, n-propyle, isopropyle, n-butyle, isobutyle, sec-butyle et tert-butyle ; l'expression "alkoxy en $C_1$-$C_4$" est équivalente à l'expression "O-alkyle en $C_1$-$C_4$" ; l'expression "acylamino en $C_2$-$C_4$" comprend notamment les groupes acétylamino et propionylamino ; le terme "halogène" vise le chlore, le brome, l'iode et le fluor ; et l'expression "noyau hétérocyclique" comprend notamment le noyau pyridyle.

Parmi les dérivés de formule (I), on préfère notamment ceux possédant les structures-particulières suivantes :

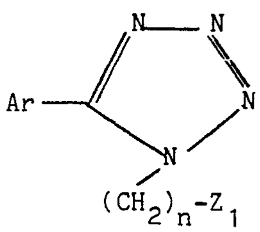 , 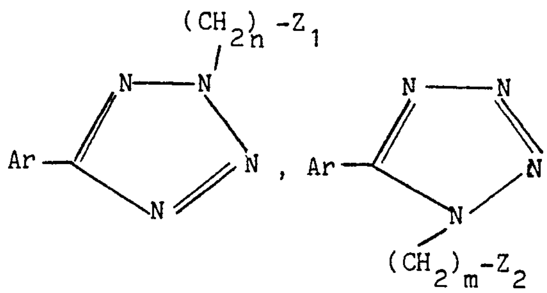 , 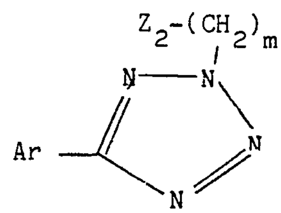

où $Z_1$ = CN, n = 1 ou 2, $Z_2$ = OH, m = entier de 2 à 6 et Ar représente un noyau phényle substitué en para par un groupe benzyloxy dont le noyau phényle est éventuellement substitué par un ou deux atomes d'halogène ou un groupe alkyle en $C_1$-$C_4$ ou alkoxy en $C_1$-$C_4$, et

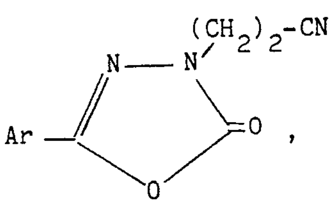 , 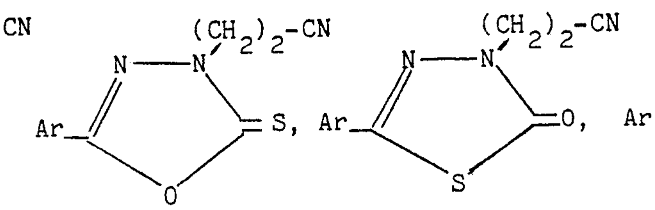 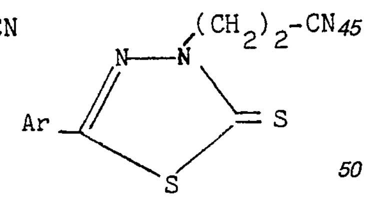

où Ar est un groupe phényle substitué en para par un groupe benzyloxy.

La présente invention s'étend en outre au procédé de préparation des dérives de formule (I).

Ce procédé comprend plus précisément :

(a) la condensation des composés de formule

$X$-$(CH_2)_n$-$z_1$ (IIa)

où n = entier de 1 à 6, $Z_1$ = CN, méthoxy ou éthoxy et X représente un groupe bon partant tel qu'un atome d'halogène ou un groupe mésyloxy ou tosyloxy, respectivement avec les composés de formule

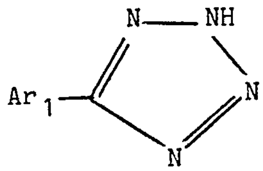

(IIIa)

où $Ar_1$ a la même signification que dans la formule (I), en présence d'une base forte, notamment une base minérale telle que KOH ou NaOH, et dans un solvant notamment alcoolique tel que le propanol, ou encore telle qu'un hydrure métallique comme l'hydrure de sodium, et dans un solvant aprotique comme le DMF, ce qui conduit aux composés de formule

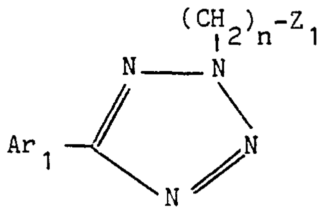

(Ia) ou

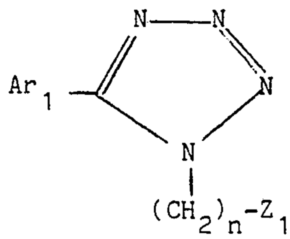

(Ib)

où $Ar_1$ a la même signification que dans la formule (I) et $(CH_2)_n$-$Z_1$ a la même signification que dans la formule (IIa) ci-dessus ;

(b) la condensation dans les mêmes conditions qu'au point (a) ci-dessus, des composés de formule

X-$(CH_2)_m$-$Z_{20}$ (IIb)

où m est un entier de 2 à 6, $Z_{20}$ représente OH, halogène, $NH_2$ substitué par deux groupes alkyle en $C_1$-$C_4$, acylamino en $C_2$-$C_4$ ou phtalimido et X a la même signification que dans la formule (IIa), respectivement avec les composés de formule

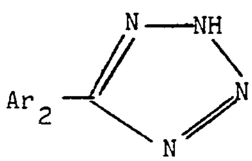

(IIIb)

où $Ar_2$ a la même signification que dans la formule (I),ce qui conduit aux composés de formule

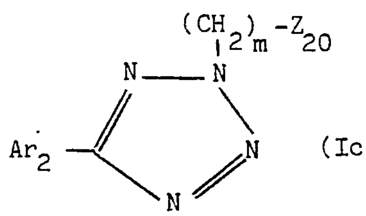

(Ic) ou

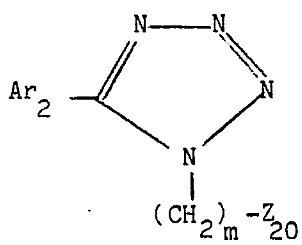

(Id)

où $Ar_2$ a la même signification que dans la formule (I) et $(CH_2)_m$-$Z_{20}$ a la même signification que dans la formule (IIb) ci-dessus;

(c) l'action de $PCl_5$ puis de l'acide hydrazoïque, en milieu aprotique comme le benzène, respectivement sur les composés de formule

$Ar_1$-CO-NH-$(CH_2)_n$-CN (IV)

où $Ar_1$ et n ont la même signification que dans la formule (I), notamment suivant la technique décrite par R. T. BUKLER dans J. Med. Chem. 13, 725 (1970), ce qui conduit aux composés de formule

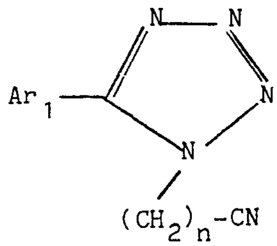

(I'b)

où $Ar_1$ et n ont la même signification que dans la formule (I) ;

(d) l'action de l'hydrazine ou d'un sel d'hydrazine comme l'acétate ou le sulfate, notamment en milieu alcoolique comme le propanol, respectivement sur les composés de formule

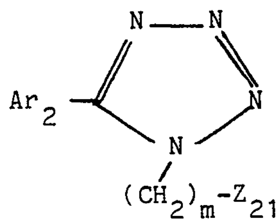

ou

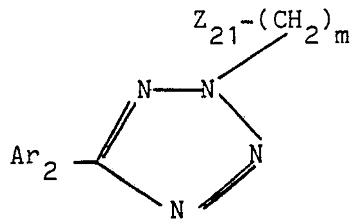

où m est un entier de 2 à 6 et $Z_{21}$ est un groupe imido tel que phtalimido et $Ar_2$ a la même signification que dans la formule (I), ce qui conduit aux composés de formule

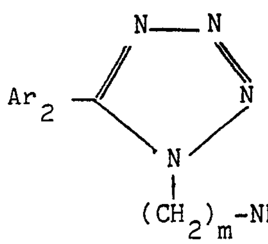

(Ie)

ou

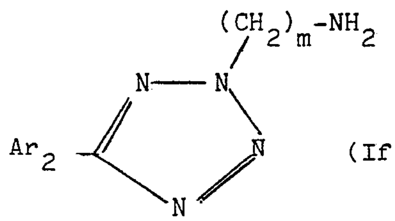

(If)

où m est un entier de 2 à 6 et $Ar_2$ a la même signification que dans la formule (I) ;

(e) la condensation, de préférence dans un solvant aprotique, sur les composés de formule (Ie) ou (If) d'un halogènure d'alkyle en $C_1$-$C_4$, notamment un iodure d'alkyle en $C_1$-$C_4$, ce qui conduit aux composés de formule

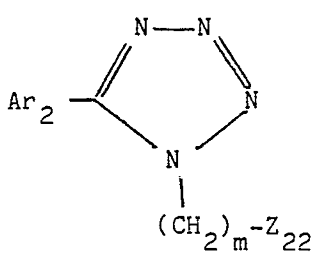

(Ig)

ou

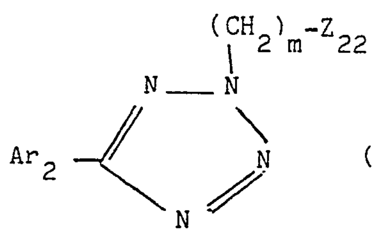

(Ih)

où m est un entier de 2 à 6 et $Z_{22} = NH_2$ substitué par un groupe alkyle en $C_1$-$C_4$ ;

(f) l'action d'une base comme l'ammoniaque par exemple, sur les composés de formule

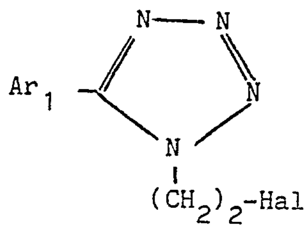

ou

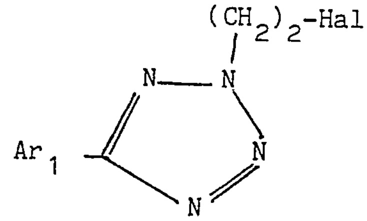

où Hal est un atome d' halogène, notamment un atome de brome, et $Ar_1$ a la même signification que dans la formule (I), ce qui conduit aux composés de formule

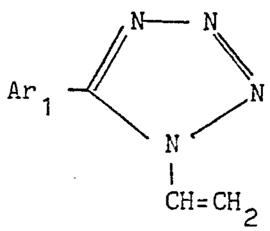 (Ii) ou 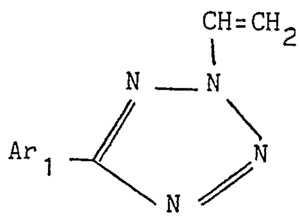 (Ij)

où $Ar_1$ a la même signification que précédemment ; ou

(g) la condensation des composés de formule

$Ar_3$-CT-NH-NH-$(CH_2)_2$-CN (V)

où $Ar_3$ a la même signification que dans la formule (I) et T est un atome d' oxygène ou de soufre, avec du phosgène ou du thiophosgène, de préférence en milieu aprotique comme le dioxanne, le chloroforme ou le toluène, ce qui conduit aux composés de formule

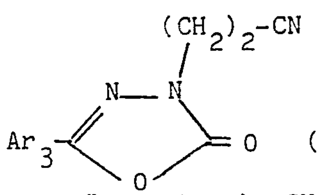 (Ik) , 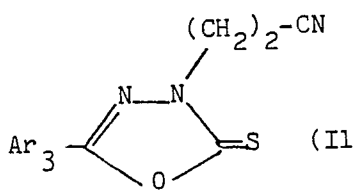 (Il)

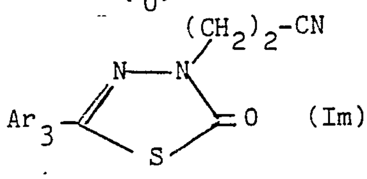 (Im) ou 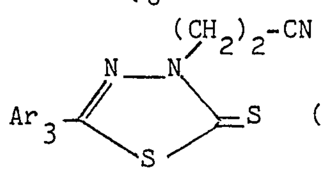 (In)

Les composés (IIIa) et (IIIb) peuvent être préparés par action de l'azoture de sodium sur les composés de formule

$Ar_1$-CN (VIa) ou $Ar_2$-CN (VIb)

de préférence selon la technique décrite par R. H Herbst dans J. Org. Chem. 22, 1142 (1957).

Les composés (VIa) ou (VIb) non connus à ce jour, à savoir ceux pour lesquels $Ar_1$ ou $Ar_2$ représente un noyau phényle substitué par un groupe benzyloxy dont le noyau phényle porte un substituant $R_1$, sont obtenus en traitant le composé de formule

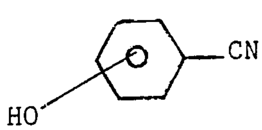

(VII)

par l'hydrure de sodium, dans un solvant aprotique tel que le DMF, puis en faisant réagir le composé ainsi obtenu respectivement avec les composés de formule

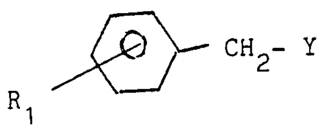 (VIII)

où Y est un groupe bon partant tel qu'un atome d'halogène ou un groupe mésyloxy ou tosyloxy.

Les composés (V) pour lesquels T est un atome d'oxygène peuvent être obtenus soit par action de la (cyano-2 éthyl) hydrazine sur les esters méthyliques ou éthyliques de l'acide de formule

$Ar_3$-COOH

en milieu alcoolique préférentiellement l'éthanol, soit par action de l'acrylonitrile en milieu alcoolique préférentiellement le propanol-1, sur les composés de formule

$Ar_3$-CO-NH-$NH_2$ (IX)

Les composés (V) pour lesquels T est un atome de soufre peuvent être obtenus par action du (cyano-2 éthyl) hydrazine sur les composés de formule

$Ar_3$-CS-S-$CH_2$-$CO_2M$ (X)

où M est un métal alcalin tel que le sodium ou le potassium.

Les composés (X) sont obtenus par action d'un acide monohalogènoacétique tel que l'acide monochloroacétique, en milieu aqueux et en présence de carbonate de sodium ou de potassium, sur les acides de formule $Ar_3$-CS-SH eux-mêmes obtenus par action du magnésien $Ar_3$-Mg-Hal où Hal est un atome d'halogène, sur le sulfure de carbone en milieu aprotique et anhydre comme le THF.

Les dérivés (I) comportant un groupe salifiable peuvent éventuellement être convertis en sels par action d'un acide organique ou minéral, dans un solvant approprié.

Les préparations suivantes sont données à titre d'exemples pour illustrer l'invention.

Exemple 1 : [(chloro-3 benzyloxy)-4 phényl]-5 tétrazole-2 propanenitrile (Ia) numéro de code : 1

1ère étape : (chloro-3 benzyloxy)-4 benzonitrile (VIa)

A une solution de $5.10^{-2}$ mole d'hydroxy-4 benzonitrile (VII) dans 100 ml de DMF, on ajoute petit à petit $5.10^{-2}$ mole de NaH de façon à obtenir une température de 25 °C. Puis la solution est chauffée à 50 °C jusqu'à cessation du dégagement d'hydrogène. Après refroidissement à 0 °C, on ajoute petit à petit $5.10^{-2}$ mole de chlorure de chloro-3 benzyle (VIII). Le mélange réactionnel est chauffé à 40 °C pendant 1 heure puis versé dans 300 ml d'eau glacée. Le solide résultant est séparé par filtration puis recristallisé dans un mélange éthanol-eau, ce qui conduit au composé attendu.

En suivant ce mode opératoire mais en mettant en oeuvre les produits de départ appropriés, on obtient les autres composés (VIa) et notamment ceux rassemblés dans le Tableau I ci-après.

2ème étape : [(chloro-3 benzyloxy)-4 phényl]-5 tétrazole (IIIa)

A une solution de $5.10^{-2}$ mole de (chloro-3 benzyloxy)-4 benzonitrile dans 20 ml de butanol, on ajoute $6{,}6.10^{-2}$ mole d' azoture de sodium et $6{,}6.10^{-2}$ mole d'acide acétique. Puis le milieu réactionnel est chauffé au reflux pendant 4 jours. On ajoute 1 g d'azoture de sodium, 2 g d'acide acétique et 10 ml de butanol et le milieu est à nouveau chauffé 2 jours au reflux. Après concentration par évaporation du solvant, le résidu est repris dans 20 ml de NaOH aqueuse à 10 %. Après filtration, la phase aqueuse est extraite à l'éther. Par acidification de la solution alcaline par HCl 2N, le composé attendu précipite et est recristallisé dans le méthanol.

Par mise en oeuvre du même mode opératoire mais à partir des composés de départ appropriés, on obtient les autres tétrazoles (IIIa) et notamment celles répertoriées dans le Tableau II ci-après.

3ème étape : [(chloro-3 benzyloxy)-4 phényl]-5 tétrazole-2 propanenitrile (Ia)

A une solution tièdie de $10^{-2}$ mole de [(chloro-3 benzyloxy)-4 phényl]-5 tétrazole dans 60 ml de n-propanol contenant $10^{-2}$ mole de KOH, on ajoute $1{,}2.10^{-2}$ mole de bromure de propionitrile (IIa) et le mélange réactionnel est chauffé au reflux pendant 25 heures. Après concentration par évaporation du solvant, le résidu est repris dans l'éther, la solution éthérée est lavée deux fois par 20 ml de NaOH aqueuse 0,5 N, puis à l'eau, séchée et concentrée. Après recristallisation dans le méthanol, on obtient le composé attendu.

Par mise en oeuvre du même mode opératoire mais à partir des réactifs appropriés, on obtient les autres composés (Ia) et notamment ceux rassemblés dans le Tableau III ci-après.

Exemple 2 : [(méthoxy-4 benzyloxy)-4 phényl]-5 tétrazole-2 éthanol [(Ic) ; numéro de code : 42] et [(méthoxy-4 benzyloxy)-4 phényl]-5 tétrazole-1 éthanol [(Id) ; numéro de code : 51]

A 30 ml de n-propanol, on ajoute $10^{-2}$ mole [(méthoxy-4 benzyloxy)-4 phényl]-5 tétrazole et $10^{-2}$ mole de bromo-2 éthanol. On chauffe au reflux pendant 25 heures. Le solvant est évaporé et le résidu est repris dans 100 ml d'éther chaud. La solution éthérée est lavée par NaOH 0,5 N, puis à l'eau. Après séchage sur sulfate de sodium, la phase éthérée est concentrée. Le composé de numéro de code 42 est obtenu après recristallisation dans le méthanol. Le solvant de recristallisation est concentré et le résidu est soumis à une chromatographie sur colonne de silice (éluant : acétate d'éthyle/éther de pétrole : 1/4) ce qui permet d'isoler

le composé de numéro de code 51.

Par mise en oeuvre du même mode opératoire mais à partir des réactifs appropriés, on obtient les composés (Ia), (Ib), (Ic) (Id) et (If) et notamment ceux rassemblés dans les Tableaux III à VI ci-après.

Exemple 3 : (diméthylamino-2 éthyl)-2 (benzyloxy-4 phényl)-5 tétrazole [(Ic) ; numéro de code : 64]

A une solution de 2,52 g (10 mmoles) de (benzyloxy-4 phényl)-5 tétrazole dans 40 ml de DMF anhydre, on ajoute 0,24 g (10 mmoles) de NaH. Après dégagement d'hydrogène et chauffage au bain-marie pendant 15 mn, la température est abaissée à 0 °C et une solution de 10 mmoles de diméthylamino-2 éthyle dans 10 ml de DMF est ajoutée. Le mélange est chauffé 2 heures 30 mn au bain-marie puis refroidi à température ambiante et versé sur de l'eau glacée. Le précipité formé est filtré, séché et recristallisé.

De la même manière mais à partir des réactifs appropriés, on obtient les composés de numéros de code 65, 66 et 67 répertoriés dans le Tableau VI ci-après.

Exemple 4 : (amino-2 éthyl)-2 (benzyloxy-4 phényl)-5 tétrazole [(If) ; numéro de code : 68]

Une solution de 2 mmoles du composé de numéro de code 67 et de 3 mmoles d'hydrate d'hydrazine, dans 20 ml de propanol-1, est chauffée au reflux pendant 3 heures 30 mn. Après refroidissement à 0 °C, le phtahydrazide (F > 300 °C) est séparé par filtration et lavé avec un peu de benzène. Le filtrat est évaporé et le résidu est recristallisé.

Exemple 5 : (benzyloxy-4 phényl)-5 vinyl-2 tétrazole [(Ij) ; numéro de code : 69]

A une suspension de 5 mmoles du composé de numéro de code 65 dans 10 ml d'ammoniaque, on ajoute de l'éthanol jusqu'à obtention d'une solution qui est agitée 2 heures à 25 °C. Le produit attendu est précipité par addition de 100 ml d'eau glacée. Après filtration et séchage, le produit est recristallisé dans l'éther de pétrole avec un rendement quantitatif.

. F = 65 °C

. IR ($\nu$ $cm^{-1}$) : 1645, 1615

. RMN (DMSO $d_6$) $\delta$ ppm : 5,15 (s, 2H) ; 5,45 (d,1H) ; 6,15 (d, 1H) ; 7,2 et 8,05 (2d, 4H ) ; 7,3 - 7,9 (sm, 5H + 1H)

TABLEAU I

$Ar_1$ - CN (VIa)

| $Ar_1$ | Rendement (%) | Point de fusion (°C) |
|---|---|---|
| (Cl-2 φ-$CH_2O$)-4 φ- | 69 | 88 |
| (Cl-3 φ-$CH_2O$)-4 φ- | 76 | 91 |
| (diCl-2,4 φ-$CH_2O$)-4 φ- | 80 | 106 |
| (diCl-2,6 φ-$CH_2O$)-4 φ- | 77 | 132 |
| ($CH_3$-4 φ-$CH_2O$)-4 φ- | 60 | 111 |
| ($CH_3$-3 φ-$CH_2O$)-4 φ- | 55 | 105 |
| ($CH_3$-2 φ-$CH_2O$)-4 φ- | 90 | 81 |
| ($CH_3O$-4 φ-$CH_2O$)-4 φ- | 83 | 130 |
| ($CH_3O$-3 φ-$CH_2O$)-4 φ- | 61 | 98 |
| (F-4 φ-$CH_2O$)-4 φ- | 78 | 120 |
| ($NO_2$-4 φ-$CH_2O$)-4 φ- | 55 | 168 |
| (I-2 φ-$CH_2O$)-4 φ- | 77 | 90 |

# TABLEAU II

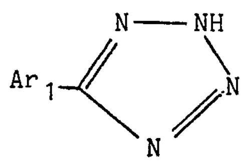

(IIIa)

| $Ar_1$ | Rendement (%) | Point de fusion (°C) |
|---|---|---|
| (ϕ-$CH_2O$)-4 ϕ- | 55 | 228 |
| (Cl-2 ϕ-$CH_2O$)-4 ϕ- | 52 | 188 |
| (Cl-3 ϕ-$CH_2O$)-4 ϕ- | 60 | 200 |
| (Cl-4 ϕ-$CH_2O$)-4 ϕ- | 61 | 240 |
| (diCl-2,4 ϕ-$CH_2O$)-4 ϕ- | 75 | 187 |
| (diCl-2,6 ϕ-$CH_2O$)-4 ϕ- | 67 | 224 |
| (diCl-3,4 ϕ-$CH_2O$)-4 ϕ- | 69 | 200 |
| ϕ - 4 ϕ- | 72 | 242 |
| ($CH_3$-4 ϕ-$CH_2O$)-4 ϕ- | 78 | 240 |
| ($CH_3$-3 ϕ-$CH_2O$)-4 ϕ- | 37 | 184 |
| ($CH_3$-2 ϕ-$CH_2O$)-4 ϕ- | 43 | 190 |
| ($CH_3O$-4 ϕ-$CH_2O$)-4 ϕ- | 30 | 220 |
| ($CH_3O$-3 ϕ-$CH_2O$)-4 ϕ- | 50 | 228 |
| (F-4 ϕ-$CH_2O$)-4 ϕ- | 80 | 204 |
| ($NO_2$-4 ϕ-$CH_2O$)-4 ϕ- | 15 | 201 |
| (I-2 ϕ-$CH_2O$)-4 ϕ- | 46 | 207 |

TABLEAU III

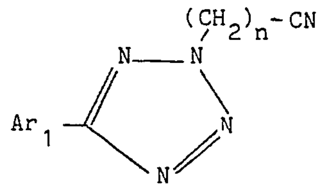

(Ia)

| N° de Code | $Ar_1$ | n | Point de fusion (°C) | Rdt (%) | Solvant de recristallisation | IR ν $cm^{-1}$ (KBr) | $H^1$ RMN δ p.p.m. (DMSO $d_6$) |
|---|---|---|---|---|---|---|---|
| 1 | (Cl-3 ϕ-$CH_2O$)-4 ϕ | 2 | 93 | 41 | Me OH | 1600-10 N=2240 - 2250 | 3,35 (t, 2H), 5,05 (t,2H), 5,3 (s, 2H) 7,1-8,2 (m, 8H) |
| 2 | (pyridyl-4)- | 2 | 166 | 50 | Ac OEt | " | 3,3 (t, 2H), 5,1 (t, 2H), 8 et 8,85 (2d, 4H) |
| 3 | ϕ- | 2 | 52 | 48 | Ac OEt/ éther de pétrole | " | 3,3 (t, 2H), 5 (t, 24), 7,35 - 8,15 (m, 5H) |
| 4 | $CH_3O$-4 ϕ- | 2 | 84 | 40 | Me OH | " | 3,3 (t, 2H), 3,85 (s, 3H)), 5,05 (,t 2H) 7,15 et 8,1 (2d, 4H) |
| 5 | $NO_2$-4 ϕ- | 2 | 162 | 52 | Me OH | " | 3,35 (t, 2H), 5,2 (t, 2H), 8,4 - 8,6 (m, 4H) |
| 6 | Cl-4 ϕ- | 2 | 100 | 55 | Me OH | " | 3,3 (t, 2H), 5,05 (t, 2H), 7,65 et 8,1 (2d, 4H) |
| 7 | (ϕ-$CH_2O$)-4 ϕ- | 2 | 128 | 61 | Me OH | " | 3,3 (t, 2H), 5 (t, 2H), 5,2 (s, 2H), 7,15 - 8,2 (m, 9H) |
| 8 | (Cl-2 ϕ-$CH_2O$)-4 ϕ | 2 | 100 | 45 | Me OH | " | 3,2 (t, 2H), 5,15 (t, 2H), 5,25 (s, 2H), 7,15 - 8,2 (m, 8H) |
| 9 | (Cl-4 ϕ-$CH_2O$)-4 ϕ | 2 | 154 | 46 | Me OH | " | 3,35 (t, 2H), 5,1 (t, 2H), 5,2 (s, 2H), 7,15 - 8,2 (m, 8H) |
| 10 | (di Cl-2,4 ϕ-$CH_2O$)-4 ϕ- | 2 | 144 | 52 | Me OH | " | 3,35 (t, 2H), 5,05 (t, 2H), 5,25 (s, 2H), 7,2 - 8,25 (m, 7,H) |
| 11 | (di Cl-2,6 ϕ-$CH_2O$)-4 ϕ- | 2 | 124 | 60 | Me OH | " | 3,3 (t, 2H), 5,05 (t, 2H), 5,25 (s, 2H), 7,2 - 8,25 (m, 7H) |
| 12 | (di Cl-3,4 ϕ-$CH_2O$)-4 ϕ- | 2 | 136 | 57 | Me OH | " | 3,3 (t, 2H), 5,05 (t, 2H), 5,25 (s, 2H), 7,15 - 8,2 (m, 7H) |
| 13 | ϕ-4 ϕ- | 2 | 134 | 56 | Me OH | " | 3,3 (t, 2H), 5 (t, 2H), 7,25 - 8,15 (m, 9H) |
| 14 | ϕ-4 ϕ- | 3 | 86 | 38 | Me OH | " | 2,1-2,75 (m, 4H), 4,8 (t, 2H) 7,35-8,35 (m, 9H) |
| 15 | ϕ-4 ϕ- | 4 | 132 | 43 | Me OH | " | 1,4-1,8 (m, 2H), 1,85-2,25 (m, 2H) 2,5 (t, 2H), 4,8 (t, 2H), 7,05 - 8,05 (m, 9H) |
| 16 | ϕ-4 ϕ- | 6 | 100 | 40 | Me OH | " | 1,1 - 1,75 (m, 6H), 1,8 - 2,15 (m, 2H), 2,4 (t, 2H), 4,7 (t, 2H), 7,3-8,3 (m, 9H) |

ϕ : 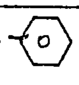

TABLEAU III (suite)

| N° de Code | $Ar_1$ | n | Point de fusion (°C) | Rdt (%) | Solvant de recristallisation | IR ν $cm^{-1}$ (KBr) | $H^1$ RMN δ p.p.m. ($DMSOd_6$) |
|---|---|---|---|---|---|---|---|
| 29 | (φ-$CH_2$O)-4 φ- | 1 | 148 | 45 | AcOEt/ éther de pétrole | 1615 | 5,2 (s, 2H) ; 6,25 (s, 2H) ; 7,1 - 7,5 (m, 7H) ; 8 (d, 2H) |
| 31 | ($CH_3$-4 φ-$CH_2$O)-4 φ- | 1 | 152 | 33 | méthanol | 1610 | 2,3 (s, 3H) ; 5,15 (s, 2H) ; 6,3 (s, 2H) ; 7,15 - 7,5 (m, 6H) ; 8,05 (d, 2H) |
| 32 | ($CH_3$-4 φ-$CH_2$O)-4 φ- | 2 | 140 | 40 | méthanol | 2255, 1610 | 2,3 (s,3H) ; 3,3 (t, 2H) ; 5,05 (t, 2H) ; 5,15 (s, 2H) ; 7,1-7,5 (m, 6H) |
| 35 | ($CH_3$-3 φ-$CH_2$O)-4 φ- | 2 | 106 | 46 | méthanol | 2250 | 2,3 (s, 3H) ; 3,3 (t, 2H) ; 5 (t, 2H) ; 5,15 (s, 2H) ; 7,05-7,45 (m, 6H) ; 8,05 (d, 2H) |
| 37 | ($CH_3$-2 φ-$CH_2$O)-4 φ- | 1 | 134 | 50 | méthanol | 1605 | 2,3 (s, 3H) ; 5,15 (s, 2H) ; 6,25 (s, 2H) ; 7,05-7,5 (m, 6H) ; 8 (d, 2H) |
| 38 | ($CH_3$-2 φ-$CH_2$O)-4 φ- | 2 | 92 | 17 | méthanol | 2240, 1605 | 2,3 (s, 3H) ; 3,3 (t, 2H) ; 5,05 (t, 2H) ; 5,15 (s, 2H) ; 7,1-7,5 (m, 6H) ; 8 (d, 2H) |
| 41 | ($CH_3$O-4 φ-$CH_2$O)-4 φ- | 2 | 118 | 10 | $CH_2Cl_2$/ éther de pétrole | 2250, 1605 | 3,3 (t, 2H) ; 3,75 (s, 3H) ; 5 (t, 2H) ; 5,1 (s, 2H) ; 6,8-7,5 (m, 6H) ; 8 (d, 2H) |

φ : 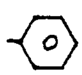

TABLEAU III (suite)

| N° de Code | $Ar_1$ | n | Point de fusion (°C) | Rdt (%) | Solvant de recristallisation | IR ν $cm^{-1}$ (KBr) | $H^1$ RMN δ p.p.m. ($DMSOd_6$) |
|---|---|---|---|---|---|---|---|
| 44 | ($CH_3O$-3 ϕ-$CH_2O$)-4 ϕ- | 2 | 109 | 34 | méthanol | 2240, 1605 | 3,75 (s, 3H) ; 3,3 (t, 2H) ; 5 (t, 2H) ; 5,15 (s, 2H) ; 6,85-7,5 (m, 6H) ; 8,05 (d, 2H) |
| 46 | (F-4 ϕ-$CH_2O$)-4 ϕ- | 1 | 144 | 60 | méthanol | 1615 | 5,15 (s, 2H) ; 6,25 (s, 2H) ; 7-7,65 (m,6H) ; 8 (d, 2H) |
| 47 | (F-4 ϕ-$CH_2O$)-4 ϕ- | 2 | 142 | 45 | méthanol | 2250, 1605 | 3,3 (t, 2H) ; 5,05 (t, 2H) ; 5,2 (s, 2H) ; 7,1-7,65 (m, 6H) ; 8,05 (d, 2H) |
| 49 | ($NO_2$-4 ϕ-$CH_2O$)-4 ϕ- | 2 | 171 | 36 | méthanol | 2240, 1610 | 3,3 (t, 2H) ; 5,3 (s, 2H) ; 5 (t, 2H) ; 5,3 (s, 2H) ; 7,2-7,7 7,8-8,2 (4d, 8H) |
| 62 | (I-2 ϕ-$CH_2O$)-4 ϕ- | 2 | 126 | 32 | méthanol | 2240 | 3,3 (t, 2H) ; 5 (t, 2H) ; 5,1 (s, 2H) ; 7,1-8,15 (m, 8H) |

ϕ : 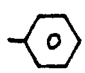

## TABLEAU IV

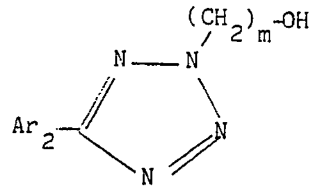

(Ic)

| N° de Code | $Ar_2$ | m | Point de fusion (°C) | Rdt (%) | Solvant de recristal-lisation | IR ν $cm^{-1}$ OH (KBr) | $H^1$ RMN δ p.p.m. ($DMSOd_6$) |
|---|---|---|---|---|---|---|---|
| 17 | (ϕ-$CH_2O$)-4 ϕ- | 2 | 113 | 63 | Me OH | 3450 | 3,9 (9,2H), 4,7 (t, 2H), 5,5-5,4 (m, 1+2H), 7,1 - 8,1 (m, 9H) |
| 18 | (Cl-2 ϕ-$CH_2O$)-4 ϕ- | 2 | 118 | 41 | Me OH | 3330 | 3,95 (q, 2H), 4,75 (t, 2H), 5,1 (t, 1H), 5,25 (s, 2H), 7,2 - 8,2 (m, 8H) |
| 19 | (Cl-3 ϕ-$CH_2O$)-4 ϕ- | 2 | 109 | 36 | Me OH | 3330 | 4 (q, 2H), 4,8 (t, 2H), 5,1 (t, 1H), 5,25 (s, 2H), 7,1 - 8,2 (m, 8H) |
| 20 | (Cl-4 ϕ-$CH_2O$)-4 ϕ- | 2 | 132 | 49 | Me OH | 3460 | 4 (q, 2H), 4,75 (t, 2H), 5,1 (t, 1H), 5,2 (s, 2H), 7,1 - 8,2 (m, 8H) |
| 21 | (di Cl-2,4 ϕ-$CH_2O$)-4 ϕ- | 2 | 135 | 58 | Me OH | 3380 | 4 (q, 2H), 4,75 (t, 2H), 5,1 (t, 1H), 5,25 (s, 2H), 7,15 - 8,25 (m, 7H) |
| 22 | (di Cl-2,6 ϕ-$CH_2O$)-4 ϕ- | 2 | 171 | 56 | Me OH | 3320 | 4 (q, 2H), 4,75 (t, 2H), 5,1 (t, 1H), 5,3 (s, 2H), 7,2 - 8,2 (m, 7H) |
| 23 | (di Cl-3,4 ϕ-$CH_2O$)-4 ϕ- | 2 | 126 | 21 | Me OH | 3280 | 4 (q, 2H), 4,75 (t, 2H), 5,1 (t, 1H), 5,2 (s, 2H), 7,1 - 8,2 (m, 7H) |
| 24 | ϕ-4 ϕ- | 2 | 148 | 52 | Ac OEt | 3460 | 3,95 (q, 2H), 4,75 (t, 2H), 5,05 (t, 1H), 7,2 - 8,1 (m, 9H) |
| 25 | ϕ-4 ϕ- | 3 | 102 | 45 | Me OH | 3300 | 1,8 - 2,1 (m, 2H), 3,8 (q, 2H), 4,6 - 4,8 (m, 1+2H), 7,3 - 8,2 (m, 9H) |
| 26 | ϕ-4 ϕ- | 4 | 105 | 48 | Me OH | 3300 | 1,3 - 1,6 (m, 2H), 1,7 - 2,1 (m, 2H), 3,45 (q, 2H), 4,5 (t, 1H), 4,75 (t, 2H), 7,4 - 8,3 (m, 9H) |
| 27 | ϕ-4 ϕ- | 6 | 102 | 40 | Me OH | 3280 | 1,15 - 1,5 (m, 6H), 1,8 - 2,1 (m, 2H), 3,35 (q, 2H), 4,25 (t, 1H), 4,65 (t, 2H), 7,2 - 8,1 (m, 9H) |
| 28 | (pyridyl-4)- | 2 | 123 | 63 | Me OH | 3190 | 3,95 (q, 2H), 4,8 (t, 2H), 5,1 (t, 1H), 8,05 et 8,8 (2d, 4H) |

ϕ : 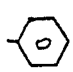

**TABLEAU IV** (suite)

| N° de Code | $Ar_2$ | m | Point de fusion (°C) | Rdt (%) | Solvant de recristallisation | IR ν $cm^{-1}$ OH (KBr) | $H^1$ RMN δ p.p.m. ($DMSOd_6$) |
|---|---|---|---|---|---|---|---|
| 33 | ($CH_3$-4 ϕ-$CH_2O$)-4 ϕ- | 2 | 125 | 60 | méthanol | 3460, 1615 | 2,6 (s, 3H) ; 3,9 (q, 2H) ; 4,75 (t, 2H) ; 5,05 (t, 1H) ; 5,15 (s, 2H) ; 7,1 - 7,5 (m, 6H) ; 8 (d,2H) |
| 36 | ($CH_3$-3 ϕ-$CH_2O$)-4 ϕ- | 2 | 100 | 17 | méthanol | 3460, 1610 | 2,3 (s, 3H) ; 3,95 (q, 2H) ; 4,75 (t, 2H) ; 5,05 (t, 1H) ; 5,15 (s, 2H) ; 6,8-7,5 (m, 6H) ; 8 (d, 2H) |
| 39 | ($CH_3$-2 ϕ-$CH_2O$)-4 ϕ- | 2 | 94,5 | 24 | méthanol | 3300, 1610 | 2,3 (s, 3H) ; 4,7 (t, 2H) ; 3,95 (q, 2H) ; 5,05 (t, 1H) ; 5,1 (s, 2H) ; 7,05-7,5 (m, 6H) ; 8 (d, 2H) |
| 42 | ($CH_3O$-4 ϕ-$CH_2O$)-4 ϕ- | 2 | 131 | 52 | méthanol | 3480, 1605 | 3,75 (s, 3H) ; 3,95 (q, 2H) ; 4,7 (t, 2H) ; 5,05 (t, 1H) ; 5,1 (s, 2H) ; 6,8-7 (m, 6H) ; 7,95 (d, 2H) |
| 45 | ($CH_3O$-3 ϕ-$CH_2O$)-4 ϕ- | 2 | 105 | 21 | méthanol | 3330, 1605 | 3,75 (s, 3H) ; 3,95 (q, 2H) ; 4,75 (t, 3H) ; 5,1 (t, 1H) ; 5,15 (s, 2H) ; 6,8-7,5 (m, 6H) 8,05 (d, 2H) |
| 48 | (F-4 ϕ-$CH_2O$)-4 ϕ- | 2 | 122 | 52 | méthanol | 3490, 1605 | 3,95 (q, 2H) ; 4,7 (t, 2H) ; 5,05 (t, 1H) ; 5,15 (s, 2H) ; 7,1-7,7 (m, 6H) ; 8,05 (d, 2H) |
| 63 | (I-2 ϕ-$CH_2O$)-4 ϕ- | 2 | 133 | 23 | méthanol | 3480 | 3,95 (q, 2H) ; 4,7 (t, 2H) ; 5,05 (t, 1H) ; 5,1 (s, 2H) ; 7,2-8,05 (m, 8H) |

ϕ : 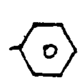

TABLEAU V

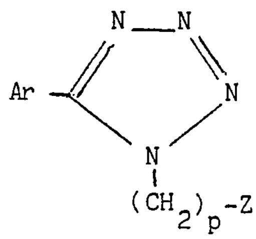

(Ib) ou (Id)

| N° de Code | Ar | p | Z | Point de fusion (°C) | Rdt (%) | Solvant de recristal-lisation | IR ν cm$^{-1}$ OH (KBr) | H$^1$ RMN δ p.p.m. (DMSOd$_6$) |
|---|---|---|---|---|---|---|---|---|
| 50 | (CH$_3$-4 ϕ-CH$_2$O)- -4 ϕ- | 1 | CN | 166 | 3 | AcOC$_2$H$_5$/ éther de pétrole | 1605 | 2,3 (s, 3H) ; 5,2 (s, 2H) ; 5,95 (s, 2H) ; 7,15-7,5 (m, 6H) ; 7,8 (d, 2H) |
| 51 | (CH$_3$O-4 ϕ-CH$_2$O)- -4 ϕ- | 2 | OH | 146 | 25 | " | 3300 1605 | |
| 52 | (F-4 ϕ-CH$_2$O)- -4 ϕ- | 1 | CN | 137 | 6 | " | 1605 | 5,2 (s, 2H) ; 5,95 (s, 2H) ; 7,3-7,8 (2d, 4H) ; 7,1-7,7 (m, 4H) |
| 53 | (F-4 ϕ-CH$_2$O)- -4 ϕ- | 2 | OH | 110 | 17 | " | 3450, 1605 | 3,85 (q, 2H) ; 4,45 (t, 2H) ; 5,1 (t, 1H) ; 5,2 (s, 2H) ; 7,1-7,65 (m, 6H) ; 7,8 (d,2H) |
| 58 | (I-2 ϕ-CH$_2$O)- -4 ϕ- | 2 | OH | 84 | 8 | " | 3400, 1605 | |
| 59 | (ϕ-CH$_2$O)-4 ϕ- | 1 | CN | 151 | 1,3 | " | 1605 | |
| 60 | (CH$_3$-2 ϕ-CH$_2$O)- -4 ϕ- | 2 | CN | 101 | 8,1 | " | 2236, 1605 | |
| 61 | (CH$_3$-3 ϕ-CH$_2$O)- -4 ϕ- | 2 | CN | 103 | 9,4 | " | 2245, 1600 | 2,35 (s, 3H) ; 3,2 (t, 2H) ; 4,75 (t, 2H) ; 5,2 (s, 2H) ; 7,1-7,4 (m, 6H) ; 7,75 (d, 2H) |

ϕ : 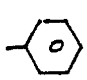

TABLEAU VI

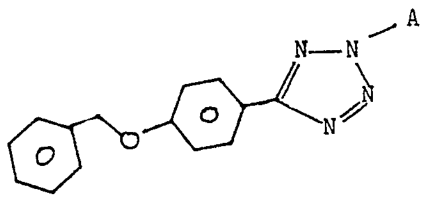

(Ia) , (Ic) ou (If)

| N° de Code | A | Point de fusion (°C) | Rdt (%) | Solvant de recristallisation | IR ν $cm^{-1}$ OH (KBr) | $H^1$ RMN δ p.p.m. ($DMSOd_6$) |
|---|---|---|---|---|---|---|
| 30 | $CH_2OCH_3$ | 90 | 20 | méthanol | 1610 | 3,4 (s, 3H) ; 5,2 (s, 2H) ; 7,1-7,6 (m, 7H) ; 8,05 (d, 2H) |
| 64 | $(CH_2)_2-N\begin{smallmatrix}CH_3\\CH_3\end{smallmatrix}$ | 67 | 40 | éther/ éther de pétrole | 1610 | 2,15 (s, 6H) ; 2,85 (t, 2H) ; 4,75 (t, 2H) ; 5,15 (s, 2H) ; 7,15 et 8 (2d, 4H) ; 7,3-7,55 ; (m, 5H) |
| 65 | $(CH_2)_2-Br$ | 110 | 70 | Ac OEt/ éther de pétrole | 1610 | 4,1 (t, 2H) ; 5,15 (s + t, 2 + 2H) 7,2 et 8,05 (2 d, 4H) ; 7,3-7,6 (m 5H) |
| 66 | $(CH_2)_2-NH-\overset{O}{\overset{\|}{C}}-CH_3$ | 142 | 30 | benzène | 3300, 1650 1610 | 1,75 (s, 3H) ; 3,65 (t, 2H) ; 4,75 (t, 2H) ; 5,15 (s, 2H) ; 7,15 et 7,95 (2d, 4H) ; 7,2-7,5 (m,5H +1H) |
| 67 | $(CH_2)_2$- 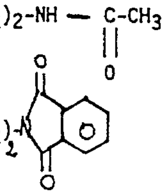 | 144 | 71 | toluène/ cyclo-hexane | 1770, 1705, 1615 | 4,1 (t, 2H) ; 5 (t, 2H) ; 5,15 (s, 2H) ; 7,2 (d, 2H) ; 7,3-7,6 (n, 5H) ; 7,85-8,1 (m, 6H) |
| 68 | $(CH_2)_2-NH_2$ | 108 | 76 | cyclohe-xane/ benzène | 3375, 3300 1615 | 2,2 (s, 2H) ; 3,15 (t, 2H) ; 4,7 (t, 2H) ; 5,2 (s, 2H) ; 7,25 et 8,1 (2d, 4H) ; 7,35-7,65 (m, 5H) |

Exemple 6 : (benzyloxy-4 phényl)-5 (cyano-2 éthyl)-3 3H-oxadiazole-1,3,4 one-2 [(Ik) ; numéro de code : 54]

1ère étape : (benzyloxy-4 benzoyl)-1 (cyano-2 éthyl)-2 hydrazine (V)

A une solution de 100 mmoles de (benzyloxy-4 benzoyl) hydrazine dans 200 ml de propanol-1, on ajoute 100 mmoles d'acrylonitrile et chauffe le mélange pendant 24 heures au reflux. Puis 50 mmoles d'acrylonitrile sont ajoutées et on poursuit le chauffage au reflux pendant 24 heures. Après refroidissement, le produit est filtré et recristallisé dans un mélange eau/éthanol pour obtenir 26 g (rdt : 88 %) du produit attendu.

. F : 136 °C

. IR (KBr) ν $cm^{-1}$ : 3290, 3220, 2240, 1670

. RMN (DMSO $d_6$)δ ppm : 2,75 (t,2H) ; 3,25 (t, 2H) ; 5,15 (s, 2H) ; 7 - 7,8 (2d, 4H) ; 7,25 - 7,6 (m, 7H)

2ème étape : (benzyloxy-4 phényl)-5 (cyano-2 éthyl)-3 3H-oxadiazole-1,3,4 one-2 (Ik)

A une solution de 10 mmoles du composé préparé à l'étape précédente, dans 100 ml de dioxanne, on ajoute goutte à goutte à 0 °C, 20 ml d'une solution toluènique à 10 % de phosgène. Ensuite, le mélange est agité 2 heures. Le composé attendu est obtenu après concentration et recristallisation dans l'éthanol avec un rendement de 77 %.

. F : 155 °C
. IR (KBr) ν $cm^{-1}$ : 2250, 1765, 1610
. RMN (DMSO $d_6$)δ ppm : 3 (t, 2H) ; 4 (t, 2H) ; 5,2 (s, 2H) ; 7 - 8 (m, 9H)

Exemple 7 : (benzyloxy-4 phényl)-5 (cyano-2 éthyl)-3 3H-oxadiazole-1,3,4 thione-2 [(Il) ; numéro de code : 55]

A une solution de 10 mmoles du composé obtenu à la première étape de l'exemple 6 dans 100 ml de chloroforme anhydre, on ajoute 10 mmoles de thiophosgène. Le mélange obtenu est chauffé 1 heure au reflux et après concentration et chromatographie sur colonne de silice (éluant : $CH_2Cl_2$) on obtient le produit attendu avec un rendement de 85 % (solvant de recristallisation : éthanol).

. F : 117 °C
. IR (KBr) ν $cm^{-1}$ : 2250, 1610
. RMN (DMSO $d_6$)δ ppm : 3,15 (t, 2H) ; 4,4 (t, 2H) ; 5,25 (t, 2H) ; 7,2 - 8,15 (m, 9H)

Exemple 8 : (benzyloxy-4 phényl)-5 (cyano-2 éthyl)-3 3H-thiadiazole-1,3,4 one-2 [(Im) ; numéro de code : 56]

1ère étape : (benzyloxy-4 thiobenzoyl)-1 (cyano-2 éthyl)-2 hydrazine (V)

Une solution de 25 ml de sulfure de carbone dans 50 ml de THF anhydre est ajoutée goutte à goutte à une solution refroidie de bromure de benzyloxy-4 phényl magnésium (préparé à partir de 200 mmoles de benzyloxy-4 bromobenzène et 6 g de magnésium dans 150 ml de THF anhydre). Le mélange est agité 12 heures à température ambiante. Après addition de 150 g de glace pilée, 25 g d'acide monochloracétique et 38 g de carbonate de sodium dans 250 ml d'eau, on laisse en agitation 1 semaine à 25 °C. Le précipité blanc formé est séparé par filtration. La phase aqueuse est acidifiée avec de l'acide sulfurique et extraite à l'éther. La phase organique est séchée sur sulfate de sodium et concentrée. On obtient l'acide dithio(benzyloxy-4 benzoyl) oxyacétique après recristallisation dans un mélange benzène/cyclohexane.

. Rdt : 52 %
. IR (ν $cm^{-1}$) : 2900, 1680

Cet acide (50 mmoles) est dissous dans une solution de NaOH à 4 % (100 ml) et 50 ml d'éthanol. Puis on ajoute 75 mmoles de (cyano-2 éthyl) hydrazine en solution dans 20 ml d'éthanol. On chauffe 15 mn à 60 - 80 °C, refroidit à 0 °C et acidifie par HCl 6N. Par filtration et purification en solution hydroalcoolique, on obtient le composé attendu avec un rendement de 38 %.

. F : 135 °C
. IR (KBr) ν $cm^{-1}$ : 3260, 3205, 3140, 2250
. RMN (DMSO $d_6$) δ ppm : 2,75 (t, 2H) ; 3,25 (t, 2H) ; 5,15 (s, 2H) ; 6,85 (s, 1H) ; 7,05 - 7,75 (2d, 4H) ; 7,25 - 7,55 (m, 6H) ; 12 (s, 1H)

2ème étape : (benzyloxy-4 phényl)-5 (cyano-2 éthyl)-3 3H-thiadiazole-1,3,4 one-2 (Im)

A une solution de 5 mmoles du composé obtenu à l'étape précédente dans 30 ml de dioxanne anhydre, on ajoute goutte à goutte en agitant à 0 °C, 7,5 ml d'une solution toluènique à 10 % de phosgène. Après 30 mn, le mélange est concentré et après recristallisation dans le butanol-1, on obtient le composé attendu avec un rendement de 59 %.

. F : 140 °C
. IR (KBr) ν $cm^{-1}$ : 2250, 1650, 1600
. RMN (DMSO $d_6$) δ ppm : 3 (t, 2H) ; 4,15 (t, 2H) ; 5,15 (s, 2H) ; 7,15 et 7, 65 (2d, 4H) ; 7,25 - 7,55 (m, 5H)

Exemple 9 : (benzyloxy-4 phényl)-5 (cyano-2 éthyl)-3 3H-thiadiazole-1,3,4 thione-2 [(In) ; numéro de code : 57]

A une solution de 5 mmoles du composé obtenu à la première étape de l'exemple 8 dans 30 ml de dioxanne anhydre, on ajoute 6 mmoles de thiophosgène, sous agitation, goutte à goutte à environ 0 °C. Après 30 mn le solvant est évaporé et le produit restant est cristallisé dans un mélange acétate d'éthyle/éther de pétrole on obtient le produit attendu avec un rendement de 51 %.

. F : 138 °C
. IR (KBr) ν $cm^{-1}$ : 2240, 1605
. RMN (DMSO $d_6$) δ ppm : 3,15 (t, 2H) ; 4,55 (t, 2H) ; 5,2 (s, 2H) ; 7,2 et 7,75 ( 2d, 4H) ; 7,3 - 7,55 (m, 5H)

Les dérivés de formule (I), leurs sels, ainsi que le phényl-5 tétrazole-2 butyronitrile et le phényl-5 tétrazole-2 valéronitrile ont été étudiés chez l'animal de laboratoire et ont montré une activité pharmacologique et notamment une activité sélective inhibitrice de la monoamine oxydase de type B (MAO-B).

L'activité inhibitrice de la monoamine oxydase de ces composés a été mise en évidence par des mesures in vitro de la MAO. Le dosage de l'activité de la MAO a été effectué en utilisant comme source d'enzymes, une suspension mitochondriale du cerveau de rat. Le dosage standard consiste à préincuber l'enzyme pendant 20 mn en absence puis en présence des inhibiteurs. Les activités sont déterminées en utilisant respectivement la sérotonine (5-HT) et la phényléthylamine (PEA) comme substrats de la MAO-A et de la MAO-B, les temps de

réaction étant de 40 mn avec la 5-HT et de 10 mn avec la PEA. Le mode opératoire mis en oeuvre est celui du protocole de P. C. Baker, Dev. Biol. 14, 267 (1966).

Les résultats exprimés en $CI_{50}$ (concentration inhibitrice 50 %) obtenus avec un certain nombre de dérivés (I) sont rassemblés dans les Tableaux VII et VIII ci-après.

TABLEAU VII

Inhibition in vitro de la monoamine oxydase

| Composé de No. de code | $CI_{50}$(MAO-A) en µM | $CI_{50}$(MAO-B) en µM |
|---|---|---|
| 1 | 150 | $2,2.10^{-3}$ |
| 7 | 86 | $2,3\ 10^{-3}$ |
| 13 | 7,8 | $840.10^{-3}$ |
| 14 | 9,6 | 1,2 |
| 16 | 46 | 5,4 |
| 18 | 88 | $5,5.10^{-3}$ |
| 20 | 60 | $8.10^{-3}$ |
| 23 | 140 | $24.10^{-3}$ |
| 27 | 80 | 16.4 |
| 54 | nd | $6,4.10^{-3}$ |
| 55 | nd | $9,3.10^{-3}$ |
| 56 | nd | $1,6.10^{-2}$ |
| 57 | nd | $6,2.10^{-2}$ |
| 30 | nd | 1,6 |
| 42 | nd | 4,7 |
| 50 | nd | $1,5.10^{-2}$ |
| 51 | nd | 0,89 |
| 32 | nd | 0,57 |

nd : n'a pas pu être déterminé, inhibition trop faible

TABLEAU VIII

Inhibition in vitro de la monoamine oxydase

| Code | $CI_{50}$ (MAO-B) en µM | % d'inhibition de MAO-A à une concentration de $10^{-6}$ M |
|---|---|---|
| 29 | 0,1 | 3 |
| 31 | 1,6 | - |
| 33 | 8 | 0 |
| 35 | 0,2 | 5 |
| 36 | 0,3 | 2 |
| 37 | 0,16 | 10 |
| 38 | 0,11 | 6 |
| 39 | 0,29 | 3 |
| 41 | 3,4 | 9 |
| 44 | 0,4 | 0 |
| 45 | 0,8 | 7 |
| 46 | 0,17 | 6 |
| 47 | 0,21 | 2 |
| 48 | 0,27 | 2 |
| 50 | 0,015 | 12 |
| 51 | 0,9 | - |
| 52 | 0,024 | 15 |
| 53 | 0,36 | 3 |
| 58 | 3,6 | 3 |
| 59 | 0,067 | 4 |
| 60 | 0,48 | 17 |
| 61 | 0,27 | 1 |
| 64 | 0,28 | 15 |
| 65 | 0,09 | 4 |
| 66 | 1,5 | 3,7 |
| 68 | 0,07 | 6 |

En ce qui concerne la toxicité des dérivés selon l'invention, on notera à titre d'exemples, qu'après administration à la souris par voie orale des composés de numéros de code 1, 7, 17 ou 19 jusqu'à une dose de 1500 mg/kg, il n'a pas été observé de toxicité pendant 24 heures.

Les dérivés (I), leurs sels pharmaceutiquement acceptables, ainsi que le phényl-5 tétrazole-2 butyronitrile et le phényl-5 tétrazole-2 valéronitrile peuvent donc être utilisés pour la préparation de médicaments inhibiteurs de la MAO-B, ces médicaments trouvant leur emploi en thérapeutique, notamment pour le traitement des troubles neurologiques liés au vieillissement pathologique, des troubles mnésiques, de l'humeur, de la schizophrénie, de la psychiasténie ou ralentissement psychique lié au vieillissement, de certaines formes de dépression et de la maladie de Parkinson.

Ces médicaments peuvent être administrés à l'homme ou à tout animal de sang chaud, sous diverses formes pharmaceutiques bien connues dans la technique considérée, et notamment sous la forme de compositions formulées en vue de leur administration par voie orale, parentérale ou rectale.

Pour l'administration par voie orale, lesdites compositions peuvent prendre la forme de comprimés, dragées ou gélules, préparés par les techniques habituelles utilisant des supports et excipients connus tels que des agents liants, des charges, des lubrifiants et des agents de désintégration ; elles peuvent également prendre la forme de solutions, sirops ou suspensions.

Pour l'administration par voie parentérale, les compositions selon l'invention peuvent prendre la forme de solutions, suspensions ou émulsions injectables comprenant un véhicule liquide, huileux ou aqueux, parentéralement acceptable.

Pour l'administration par voie rectale, les compositions peuvent prendre la forme de suppositoires comprenant les bases habituelles pour suppositoires.

La dose thérapeutiquement active des principes actifs, c'est-à-dire des dérivés (I), de leurs sels pharmaceutiquement acceptables, ainsi que du phényl-5 tétrazole-2 butyronitrile et du phényl-5 tétrazole-2 valéronitrile, dépend notamment de la voie d'administration, du poids corporel du patient et de la puissance thérapeutique des principes actifs mis en oeuvre. Généralement, par voie orale, les doses administrées pourront atteindre 600 mg de principe actif par jour (en une ou plusieurs prises) ; par voie parentérale, elles pourront atteindre 100 mg de principe actif par jour (en une ou plusieurs injections journalières) ; et par voie

rectale, elles pourront atteindre 300 mg de principe actif par jour (en un ou plusieurs suppositoires).

**Revendications**

1. Dérivés de formule :

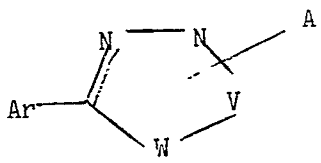

(I)

dans laquelle l'enchaînement

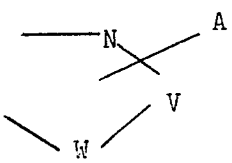

représente

1) soit l'enchaînement de structure :

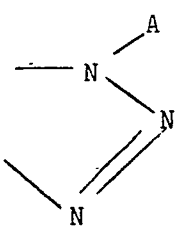

ou

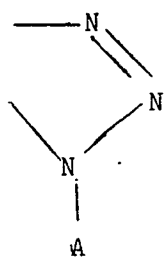

où A est :

a) un groupe vinyle ou un groupe $(CH_2)_n$-$Z_1$ dans laquelle $Z_1$ représente un groupe CN, méthoxy ou éthoxy et n est un nombre entier de 1 à 6, auquel cas Ar prend la valeur $Ar_1$ qui représente :

. un noyau phényle,

. un noyau phényle substitué par un ou deux atomes d'halogène ou un groupe $NO_2$, méthyle, éthyle, méthoxy, éthoxy, phényle ou benzyloxy de formule :

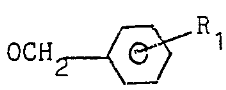

où $R_1$ représente un atome d hydrogène, un ou deux atome d' halogène ou un groupe CN, $NO_2$, $NH_2$, $CF_3$, alkyle en $C_1$-$C_4$, alkoxy en $C_1$-$C_4$ ou acylamino en $C_2$-$C_4$, ou

. un noyau hétérocyclique ; ou

b) un groupe de formule $(CH_2)_m$-$Z_2$ dans laquelle $Z_2$ représente un groupe OH, un atome d'halogène, un groupe $NH_2$, un groupe $NH_2$ substitué par un ou deux groupes alkyle en $C_1$-$C_4$, un groupe acylamino en $C_2$-$C_4$ ou un groupe phtalimido et m est un nombre entier de 2 à 6, auquel cas Ar prend la valeur $Ar_2$ qui représente :

. un noyau phényle substitué par un groupe alkoxy en $C_1$-$C_4$ ou benzyloxy de formule

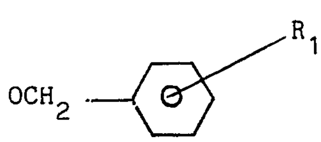

où $R_1$ a la même signification que ci-dessus, ou

. un noyau hétérocyclique ;

2) soit l'enchaînement de structure

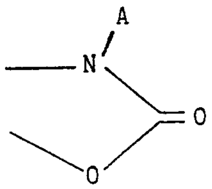 , 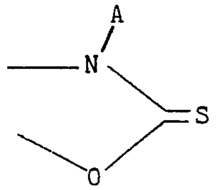 , 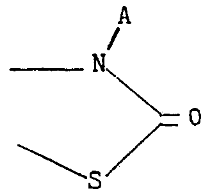 ou 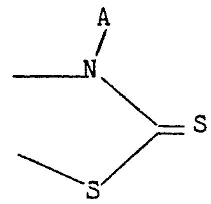

où A est un groupe cyano-2 éthyle, auquel cas Ar prend la valeur $Ar_3$ qui représente un noyau phényle substitué par un groupe alkyle en $C_1$-$C_4$, alkoxy en $C_1$-$C_4$, phényle ou benzyloxy de formule

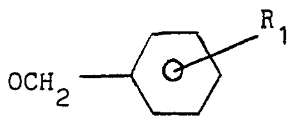

où $R_1$ a la même signification que ci-dessus,
ainsi que les sels d'addition d'acide de ces dérivés (I) comportant un groupe salifiable, à l'exclusion du phényl-5 tétrazole-2 butyronitrile et du phényl-5 tétrazole-2 valéronitrile.

2. Dérivés selon la revendication 1, de structure :

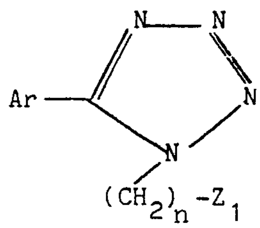 , 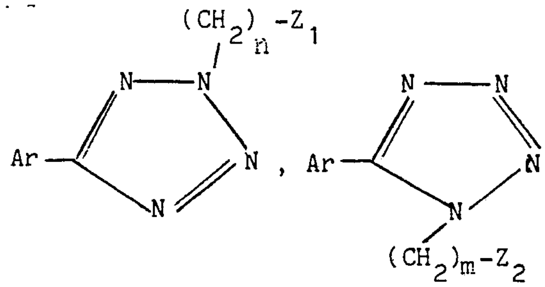 , 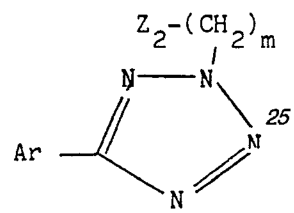

où $Z_1$ = CN, n = 1 ou 2, $Z_2$ = OH, m = entier de 2 à 6 et Ar représente un noyau phényle substitué en para par un groupe benzyloxy dont le noyau phényle est éventuellement substitué par un ou deux atomes d'halogène ou un groupe alkyle en $C_1$-$C_4$ ou alkoxy en $C_1$-$C_4$.

3. Dérivés selon la revendication 1, de structure :

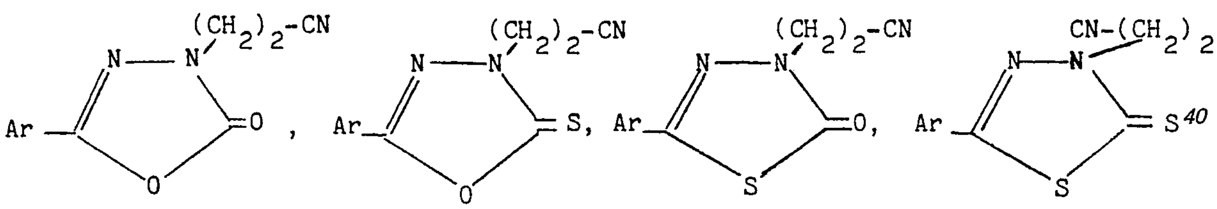

où Ar est un groupe phényle substitué en para par un groupe benzyloxy.

4. Procédé de préparation des dérivés de formule (I) selon la revendication 1, caractérisé en ce qu'il comprend :

(a) la condensation des composés de formule

$X-(CH_2)_n-Z_1$ (IIa)

où n = entier de 1 à 6, $Z_1$ = CN, méthoxy ou éthoxy et X représente un groupe bon partant tel qu'un atome d'halogène ou un groupe mésyloxy ou tosyloxy, respectivement avec les composés de formule

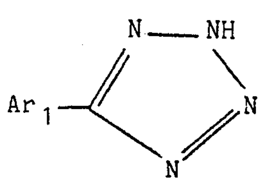 (IIIa)

où $Ar_1$ a la même signification que dans la formule (I), en présence d'une base forte, ce qui conduit aux composés de formule

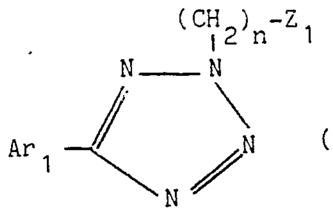 (Ia) ou 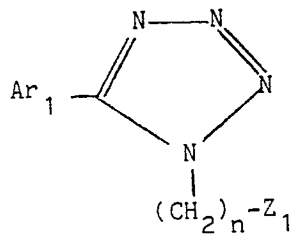 (Ib)

où $Ar_1$ a la même signification que dans la formule (I) et $(CH_2)_n$-$Z_1$ a la même signification que dans la formule (IIa) ci-dessus ;

(b) la condensation des composés de formule

$Z$-$(CH_2)_m$-$Z_{20}$ (IIb)

où m est un entier de 2 à 6, $Z_{20}$ représente OH, halogène, $NH_2$ substitué par deux groupes alkyle en $C_1$-$C_4$, acylamino en $C_2$-$C_4$ ou phtalimido et X a la même signification que dans la formule (IIa), respectivement avec les composés de formule

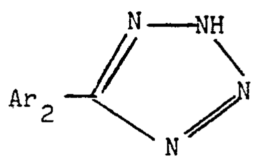 (IIIb)

où $Ar_2$ a la même signification que dans la formule (I),ce qui conduit aux composés de formule

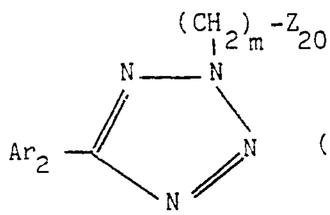 (Ic) ou 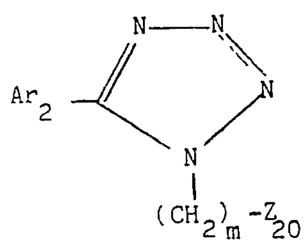 (Id)

où $Ar_2$ a la même signification que dans la formule (I) et $(CH_2)_m$-$Z_{20}$ a la même signification que dans la formule (IIb) ci-dessus ;

(c) l'action de $PCl_5$ puis de l'acide hydrazoïque respectivement sur les composés de formule

$Ar_1$-CO-NH-$(CH_2)_n$-CN (IV)

où $Ar_1$ et n ont la même signification que dans la formule (I), ce qui conduit aux composés de formule

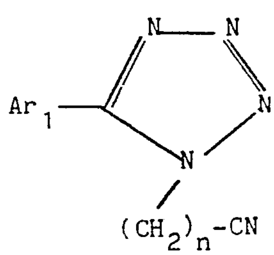 (I'b)

où $Ar_1$ et n ont la même signification que dans la formule (I) ;

(d) l'action de l'hydrazine ou d'un sel d'hydrazine respectivement sur les composés de formule

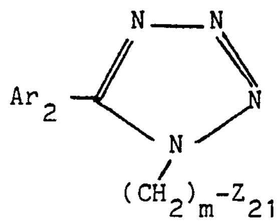 ou 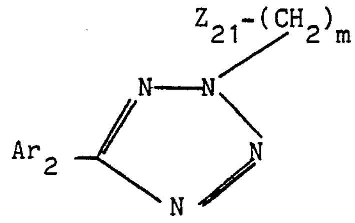

où m est un entier de 2 à 6 et $Z_{21}$ est un groupe imido et $Ar_2$ a la même signification que dans la formule (I), ce qui conduit aux composés de formule

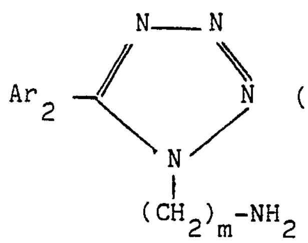 (Ie) ou 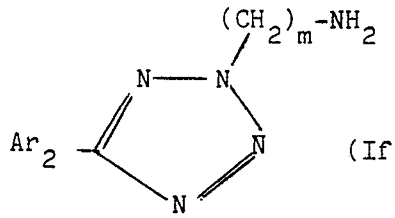 (If)

où m est un entier de 2 à 6 et $Ar_2$ a la même signification que dans la formule (I) ;

(e) la condensation sur les composés de formule (Ie) ou (If) d'un halogènure d alkyle en $C_1$-$C_4$, ce qui conduit aux composés de formule

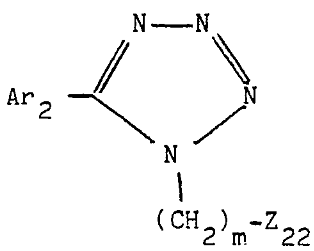 (Ig) ou 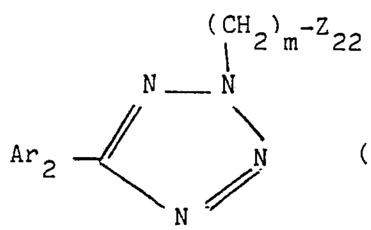 (Ih)

où m est un entier de 2 à 6 et $Z_{22}$ = $NH_2$ substitué par un groupe alkyle en $C_1$-$C_4$ ;

(f) l'action d'une base comme l'ammoniaque sur les composés de formule

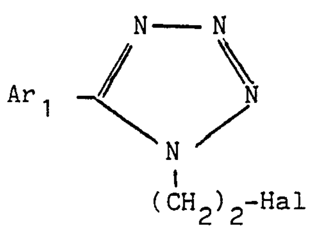 ou 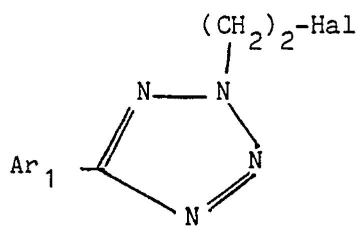

où Hal est un atome d'halogène et $Ar_1$ a la même signification que dans la formule (I), ce qui conduit aux composés de formule

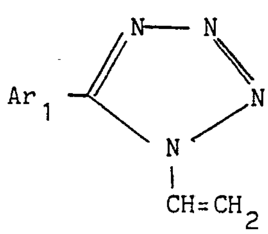 (Ii) ou 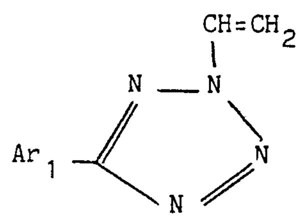 (Ij)

où $Ar_1$ a la même signification que précédemment ; ou

(g) la condensation des composés de formule

$Ar_3$-CT-NH-NH-$(CH_2)_2$-CN (V)

où $Ar_3$ a la même signification que dans la formule (I) et T est un atome d' oxygène ou de soufre, avec du phosgène ou du thiophosgène, ce qui conduit aux composés de formule

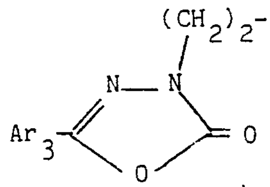 (Ik) , 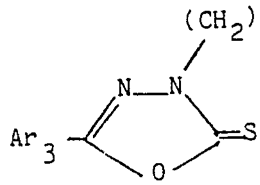 (Il)

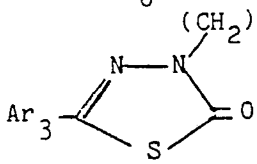 (Im) ou 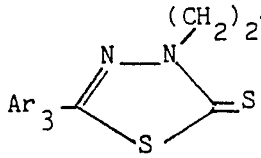 (In)

5. Composition pharmaceutique, caractérisée en ce qu'elle comprend un dérivé de formule (I) selon la revendication 1, un sel pharmaceutiquement acceptable de ce dérivé, le phényl-5 tétrazole-2 butyronitrile ou le phényl-5 tétrazole-2 valéronitrile et un support ou un excipient pharmaceutiquement acceptable.

6. Utilisation des dérivés de formule (I) selon la revendication 1, des sels pharmaceutiquement acceptables de ces dérivés, du phényl-5 tétrazole-2 butyronitrile ou du phényl-5 tétrazole-2 valéronitrile pour la préparation d'un médicament inhibiteur de la monoamine oxydase de type B.

7. Composés de formule

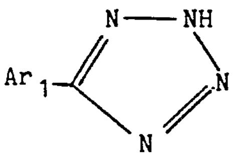 (IIIa)

où $Ar_1$ représente :

. un noyau phényle,

. un noyau phényle substitué par un ou deux atomes d'halogène ou un groupe $NO_2$, méthyle, éthyle, méthoxy, éthoxy, phényle ou benzyloxy de formule :

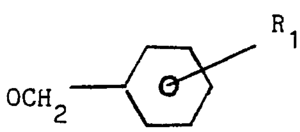

où $R_1$ représente un atome d hydrogène, un ou deux atome d'halogène ou un groupe CN, $NO_2$, $NH_2$, $CF_3$, alkyle en $C_1$-$C_4$, alkoxy en $C_1$-$C_4$ ou acylamino en $C_2$-$C_4$, ou

. un noyau hétérocyclique.

8. Composés de formule

$Ar_1$-CN (VIa)

où $Ar_1$ représente un noyau phényle substitué par un groupe benzyloxy dont le reste phényle est éventuellement substitué par un ou deux atomes d'halogène ou un groupe CN, $NO_2$, $NH_2$, $CF_3$, alkyle en $C_1$-$C_4$, alkoxy en $C_1$-$C_4$ ou acylamino en $C_2$-$C_4$.

9. Composés de formule

$Ar_3$-CT-NH-NH-$(CH_2)_2$-CN (V)

où T est un atome d'oxygène ou de soufre et $Ar_3$ représente un noyau phényle substitué par un groupe alkyle en $C_1$-$C_4$, alkoxy en $C_1$-$C_4$, phényle ou benzyloxy dont le noyau phényle est éventuellement substitué par un ou deux atomes d'halogène ou un groupe CN, $NO_2$, $NH_2$, $CF_3$, alkyle en $C_1$-$C_4$, alkoxy en $C_1$-$C_4$ ou acylamino en $C_2$-$C_4$.

**Revendications pur les Etats contractants suivants: AT, ES, GR**

1. Procédé de préparation des dérivés de formule :

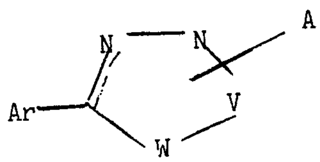

(I)

dans laquelle l'enchaînement

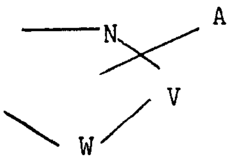

représente

1) soit l'enchaînement de structure :

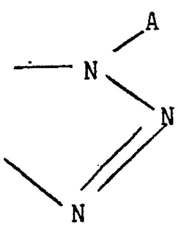

ou

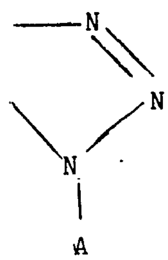

où A est :

a) un groupe vinyle ou un groupe $(CH_2)_n$-$Z_1$ dans laquelle $Z_1$ représente un groupe CN, méthoxy ou éthoxy et n est un nombre entier de 1 à 6, auquel cas Ar prend la valeur $Ar_1$ qui représente :

. un noyau phényle,

. un noyau phényle substitué par un ou deux atomes d'halogène ou un groupe $NO_2$, méthyle, éthyle, méthoxy, éthoxy, phényle ou benzyloxy de formule :

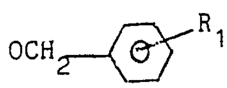

où $R_1$ représente un atome d'hydrogène, un ou deux atome d'halogène ou un groupe CN, $NO_2$, $NH_2$, $CF_3$, alkyle en $C_1$-$C_4$, alkoxy en $C_1$-$C_4$ ou acylamino en $C_2$-$C_4$, ou

. un noyau hétérocyclique ; ou

b) un groupe de formule $(CH_2)_m$-$Z_2$ dans laquelle $Z_2$ représente un groupe OH, un atome d'halogène, un groupe $NH_2$, un groupe $NH_2$ substitué par un ou deux groupes alkyle en $C_1$-$C_4$, un groupe acylamino en $C_2$-$C_4$ ou un groupe phtalimido et m est un nombre entier de 2 à 6, auquel cas Ar prend la valeur $Ar_2$ qui représente :

. un noyau phényle substitué par un groupe alkoxy en $C_1$-$C_4$ ou benzyloxy de formule

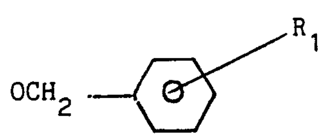

où $R_1$ a la même signification que ci-dessus, ou

. un noyau hétérocyclique ;

2) soit l'enchaînement de structure

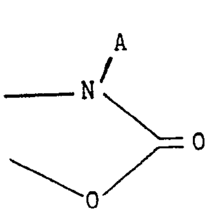 , 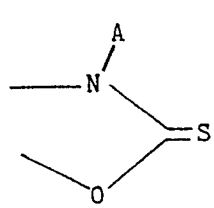 , 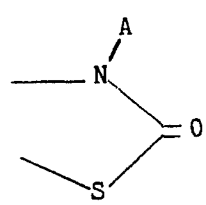 ou 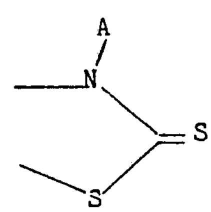

où A est un groupe cyano-2 éthyle, auquel cas Ar prend la valeur $Ar_3$ qui représente un noyau

phényle substitué par un groupe alkyle en $C_1$-$C_4$, alkoxy en $C_1$-$C_4$, phényle ou benzyloxy de formule

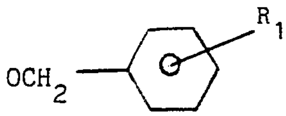

où $R_1$ a la même signification que ci-dessus,
à l'exclusion du phényl-5 tétrazole-2 butyronitrile et du phényl-5 tétrazole-2 valéronitrile,
caractérisé en ce qu'il comprend :

(a) la condensation des composés de formule

$X-(CH_2)_n-Z_1$ (IIa)

où n = entier de 1 à 6, $Z_1$ = CN, méthoxy ou éthoxy et X représente un groupe bon partant tel qu'un atome d'halogène ou un groupe mésyloxy ou tosyloxy, respectivement avec les composés de formule

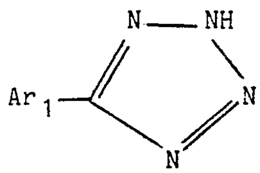

(IIIa)

où $Ar_1$ a la même signification que dans la formule (I), en présence d'une base forte, ce qui conduit aux composés de formule

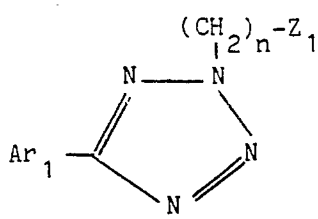

(Ia) ou

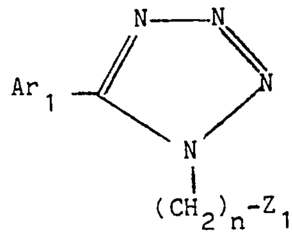

(Ib)

où $Ar_1$ a la même signification que dans la formule (I) et $(CH_2)_n-Z_1$ a la même signification que dans la formule (IIa) ci-dessus ;

(b) la condensation des composés de formule

$X-(CH_2)_m-Z_{20}$ (IIb)

où m est un entier de 2 à 6, $Z_{20}$ représente OH, halogène, $NH_2$ substitué par deux groupes alkyle en $C_1$-$C_4$, acylamino en $C_2$-$C_4$ ou phtalimido et X a la même signification que dans la formule (IIa), respectivement avec les composés de formule

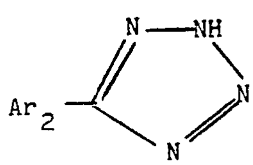

(IIIb)

où $Ar_2$ a la même signification que dans la formule (I),ce qui conduit aux composés de formule

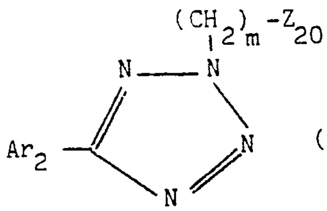

(Ic) ou

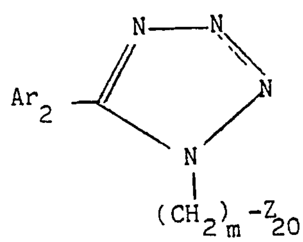

(Id)

où $Ar_2$ a la même signification que dans la formule (I) et $(CH_2)_m-Z_{20}$ a la même signification que dans la formule (IIb) ci-dessus ;

(c) l'action de $PCl_5$ puis de l'acide hydrazoïque respectivement sur les composés de formule

$Ar_1$-CO-NH-$(CH_2)_n$-CN (IV)

où $Ar_1$ et n ont la même signification que dans la formule (I), ce qui conduit aux composés de formule

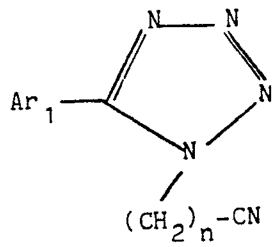

(I'b)

où $Ar_1$ et n ont la même signification que dans la formule (I) ;

(d) l'action de l'hydrazine ou d'un sel d'hydrazine respectivement sur les composés de formule

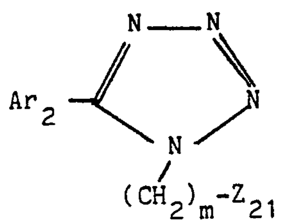

ou

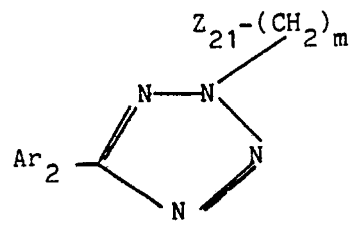

où m est un entier de 2 à 6 et $Z_{21}$ est un groupe imido et $Ar_2$ a la même signification que dans la formule (I), ce qui conduit aux composés de formule

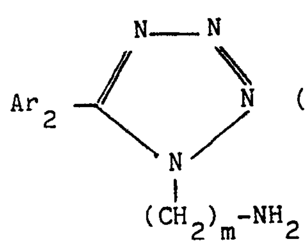

(Ie) ou

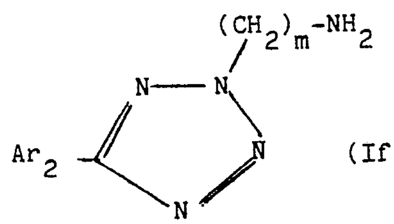

(If)

où m est un entier de 2 à 6 et $Ar_2$ a la même signification que dans la formule (I) ;

(e) la condensation sur les composés de formule (Ie) ou (If) d'un halogènure d'alkyle en $C_1$-$C_4$, ce qui conduit aux composés de formule

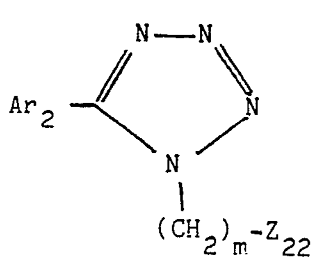

(Ig) ou

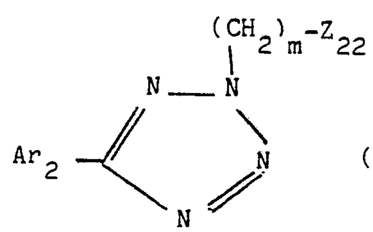

(Ih)

où m est un entier de 2 à 6 et $Z_{22}$ = $NH_2$ substitué par un groupe alkyle en $C_1$-$C_4$ ;

(f) l'action d'une base comme l'ammoniaque sur les composés de formule

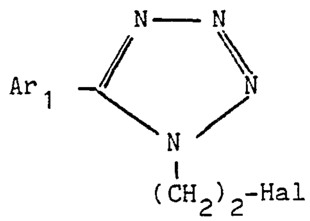 ou 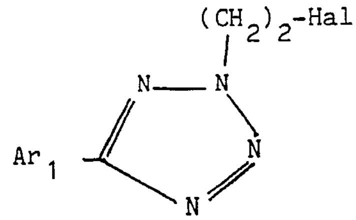

où Hal est un atome d'halogène et $Ar_1$ a la même signification que dans la formule (I), ce qui conduit aux composés de formule

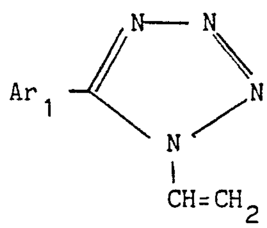 (Ii) ou 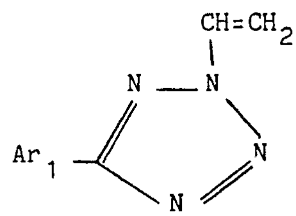 (Ij)

où $Ar_1$ a la même signification que précédemment ; ou

(g) la condensation des composés de formule

$Ar_3-CT-NH-NH-(CH_2)_2-CN$ (V)

où $Ar_3$ a la même signification que dans la formule (I) et T est un atome d'oxygène ou de soufre, avec du phosgène ou du thiophosgène, ce qui conduit aux composés de formule

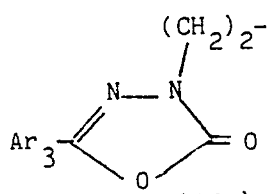 (Ik) , 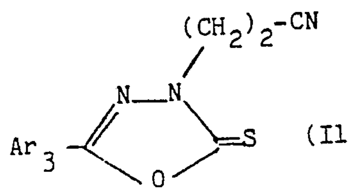 (Il)

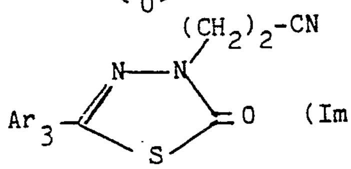 (Im) ou 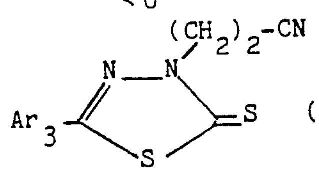 (In)

2. Procédé selon la revendication 1 pour la préparation des dérivés de structure :

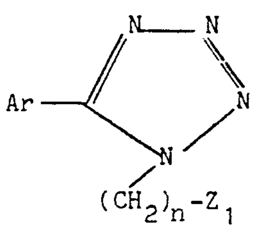 , 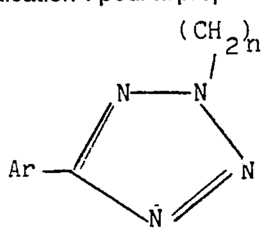 , 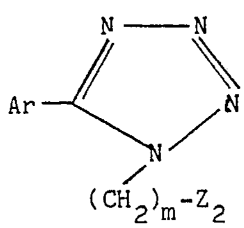 , 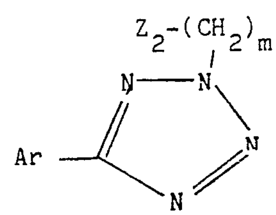

où $Z_1$ = CN, n = 1 ou 2, $Z_2$ = OH, m = entier de 2 à 6 et Ar représente un noyau phényle substitué en para par un groupe benzyloxy dont le noyau phényle est éventuellement substitué par un ou deux atomes d'halogène ou un groupe alkyle en $C_1$-$C_4$ ou alkoxy en $C_1$-$C_4$.

3. Procédé selon la revendication 1 pour la préparation des dérivés de structure :

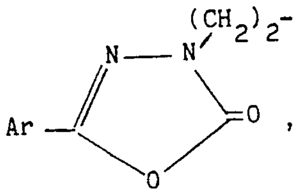 , 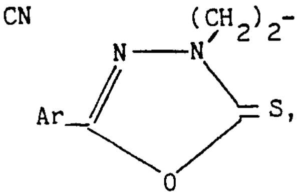 , 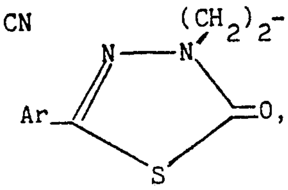 , 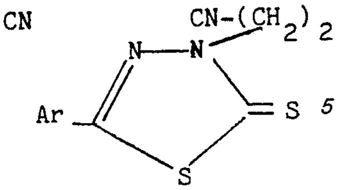

où Ar est un groupe phényle substitué en para par un groupe benzyloxy.

4. Utilisation des dérivés de formule (I) définie à la revendication 1, des sels pharmaceutiquement acceptables de ces dérivés, du phényl-5 tétrazole-2 butyronitrile ou du phényl-5 tétrazole-2 valéronitrile pour la préparation d'un médicament inhibiteur de la monoamine oxydase de type B.